**Europäisches Patentamt**

**European Patent Office**

**Office européen des brevets**

(19)

(11) Publication number:

**0 251 579**
**A2**

# EUROPEAN PATENT APPLICATION

(21) Application number: 87305404.3

(22) Date of filing: 18.06.87

(51) Int. Cl.⁴: **C12N 15/00 , A61K 39/106**

(30) Priority: 24.06.86 AU 6553/86

(43) Date of publication of application:
07.01.88 Bulletin 88/01

(84) Designated Contracting States:
**AT BE CH DE ES FR GB GR IT LI LU NL SE**

(71) Applicant: **ENTEROVAX RESEARCH PTY. LTD.**
**University of Adelaide**
**North Terrace Adelaide South Australia(AU)**

(72) Inventor: **Morona, Renato**
**10 Talbot Road**
**Waterloo Corner South Australia(AU)**
Inventor: **Manning, Paul A.**
**19 Elgin Way**
**Flagstaff Hill South Australia(AU)**

(74) Representative: **Harding, Richard Patrick et al**
**Arthur R. Davies & Co. 27 Imperial Square**
**Cheltenham GL50 1RQ(GB)**

(54) **Non-antibiotic marker system.**

(57) A plasmid bearing a non-antibiotic member including a suitable plasmid cloning vector; and a first cloned fragment of DNA containing a non-reverting thyA⁺ gene attached thereto.

EP 0 251 579 A2

## "Non-Antibiotic Marker System"

The present invention relates to a non-antibiotic marker for use in plasmid manipulation.

It is known to utilise the antibiotic resistance encoded on a plasmid or bacterial strain in order to select for the maintenance of a plasmid or the like in a strain during plasmid manipulations. Whilst antibiotic resistance provides a powerful marker system, there are substantial disadvantages related thereto. Firstly, as most antibiotics are in clinical use, the use of a resistant organism reduces the choice in treating a disease related to the bacterial strain of interest. For this reason, some regulatory authorities will not permit the use of antibiotic resistance as a selection marker.

Attempts have been made to utilise non-antibiotic resistance, for example mercury (Hg) resistance as a selection marker. However this technique has numerous difficulties which include the generation of volatile mercury during expression. This creates substantial difficulties in cleaning the apparatus and represents a pollution problem.

A second problem with the utilisation of antibiotic resistance as a selection marker is that the system requires the presence of an antibiotic whenever the organism is grown. This requirement is a substantial complication in the growth process and adds considerably to its expense. Moreover, in some circumstances, the antibiotic resistance may be due to the expression of an enzyme which degrades the antibiotic. For example, ampicillin resistance is due to the expression of the enzyme b-lactamase which cleaves the antibiotic. Thus it is necessary to continually add antibiotic to a growing culture in order to maintain the selection.

Accordingly, it is an object of the present invention to overcome, or at least alleviate, one or more of the difficulties related to the prior art.

Accordingly, in a first aspect, there is provided a method for preparing a plasmid including a non-antibiotic marker including

providing

(a) a thyA+ gene fragment, and

(b) a suitable plasmid cloning vector, and inserting the thyA+ gene fragment into a suitable site on the plasmid. The plasmid may include at least one antibiotic resistance coding region. The antibiotic resistance may be a tetracycline resistance or ampicillin resistance. Accordingly the method according to this aspect of the present invention may further include

(c) treating the plasmid bearing the thyA + gene to inactivate at least one antibiotic resistance segment.

In a preferred form, the second and third steps may be combined by inserting the thyA+ gene fragment into the plasmid at a site whithin at least one antibiotic resistance coding region to eliminate the antibiotic resistance.

It will be understood that where there is more than one segment of antibiotic resistance in the plasmid, step (ii) may eliminate one segment coding for antibiotic resistance and step (iii) another.

The thyA+ gene fragment for use in the preparation of the plasmid according to this aspect of the present invention may be obtained in any suitable form. The Escherichia coli K12 gene encoding the enzyme thymidylate synthetase (thyA+) is particularly suitable since it is located on a small DNA fragment (1.2kb HindIII-HindIII) and is able to complement chromosome mutations in this gene. The thyA+ gene is very conserved and is found in bacteriophages, prokaryotes and eukaryotes. Because the enzyme is conserved, the cloned thyA+ gene from, for example, E Coli K-12 is able to complement the mutant thyA genes located in the chromosome of related bacterial species as discussed below. The fragment bearing the thyA + gene may be isolated from E coli K12. Alternatively, the source of the thyA+ gene may be a suitable plasmid. A plasmid pBTAH has been found to be suitable.

The gene fragment may be isolated from the plasmid and inserted into and replace the or one of the antibiotic resistance marker(s) of a plasmid. The plasmid may in turn carry cloned genes of interest. Alternatively, the fragment may be inserted into a plasmid which is then used as a cloning vector for introduction of the cloned genes of interest.

The plasmid cloning vector utilised in the method according to the present invention may be of any suitable type. The plasmid cloning vector may be selected from the plasmids pSC101, pUC18, pUC19, pBR322 and pBTAH.

In a further aspect of the present invention, there is provided a plasmid including

(a) a suitable plasmid cloning vector

(b) a first cloned fragment of DNA containing a non-reverting thyA+ gene as a non-antibiotic selection marker inserted into the plasmid vector. The plasmid may include no regions coding for antibiotic resistance.

Further an antibiotic resistance coding region on the plasmid coding vector is inactivated by insertion of the cloned fragment of DNA containing a thyA+ gene or is otherwise inactivated.

(c) at least one further cloned fragment of DNA including a gene of interest. The gene of interest may be a gene determinant of disease of importance in humans and/or animals. They may accordingly be of importance in disease control. For example, the further fragment on the plasmid may contain the ompV gene which is a gene which expresses an outer membrane protein of Vibrio cholerae, the cause of the disease, cholera, in man. As the ompV gene may express a protective antigen, it is of interest in the preparation of anticholera vaccines.

In another example, the further fragment on the plasmid may contain a gene expressing the O-antigen. The O-antigen is part of the lipopolysaccharide on cell surfaces. Accordingly, it is of interest in preparation of vaccines for the treatment of cholera in man.

Particular plasmids of interest are plasmids designated pEVX1, pEVX2, pEVX3, pEVX5, pEVX8 and pEVX9, samples of which are maintained in the culture collection of the University of Adelaide, Australia.

The plasmid pEVX1 was constructed, briefly, as follows:

(1) An EcoRI - HindIII fragment from pOmpV210 was sub-cloned into the ScaI site of pBR322. This fragment contains the ompV gene and the resulting plasmid, designated pOmpV500, expresses the OmpV protein.

(2) A HindIII - HindIII fragment from the plasmid pBTAH, which contains the thyA+ gene, was sub-cloned into the HindIII site of pOmpV500. The resulting plasmid was called pOmpV501.

(3) A deletion removing most of the tetracycline resistance coding region of pOmpV501 was created by digesting the plasmid with NaeI and re-ligating the fragments. The resulting plasmid had lost all it's NaeI restriction sites. This plasmid was called pEVX1 and expresses OmpV protein and is capable of complementing thyA mutants.

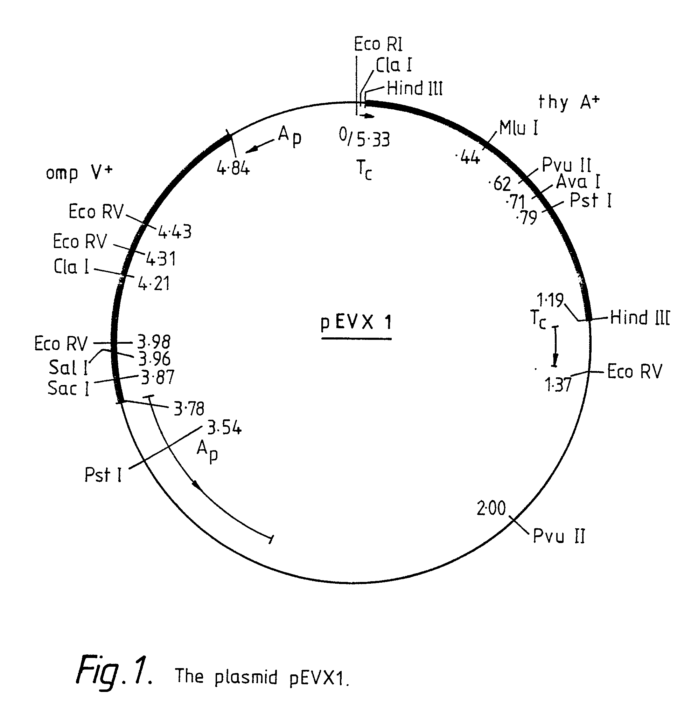

Fig. 1 below provides a restriction map of the plasmid pEVX1. Accordingly in a preferred aspect of the present invention, there is provided the plasmid, pEVXl.

Also of interest in this aspect are plasmids pEVX2, pEVX3, pEVX5, pEVX8, pEVX9, pEVX13, pEVX14, pEVX17, pEVX18, pEVX21, pEVX22, pEVX23, pEVX24, pEVX25, pEVX30, pEVX31, pEVX33 and pEVX37.

The plasmids pEVX2 and pEVX3 were made as follows:

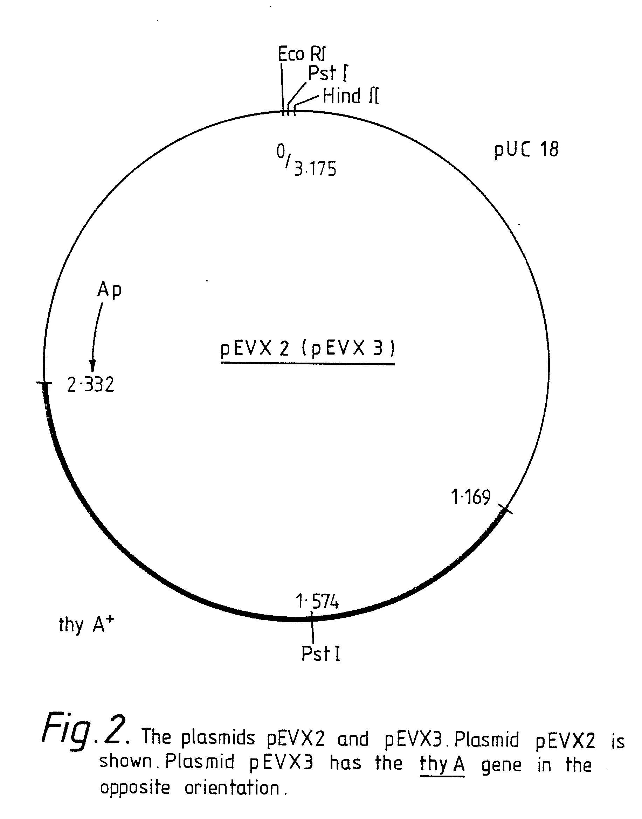

The base plasmid here was the high copy number polylinker-containing pUC18. The ampicillin resistance gene of pUC18 was deleted by digesting the plasmid with DraI: this enzyme results in blunt-ends being formed. The 1.2kb HindIII thyA+ piece from pBTAH was end-filled with DNA polymerase (Klenow fragment) and subsequently blunt-end ligated to the DraI digested pUC18. The ligated DNA was transformed into strain EX98 (E.coli K12 thyA) and thyA + transformants were selected. Transformants were screened for the type of plasmid they contained. Plasmids containing the thyA+ gene in either orientation were found. Two such plasmids are pEVX2 and pEVX3. They are shown in Figure 2.

The plasmid pEVX5 was made as follows:

We sought to make a low copy number thyA + vector with a unique SacI site. The plasmid would be useful for subcloning of the genes encoding the O-antigen from Vibrio cholerae 017. The well known plasmid pSC101 was chosen since it can be mobilized, albeit very poorly, by E. coli K-12 Hfr donor strains.

The unique SacI site was introduced into pSC101 as follows: pSC101 was digested with SmaI, and subsequently SacI linkers were ligated onto the SmaI blunt ends. After digesting with SacI, the plasmids were re-circularized and transformed into strain DH1 (E.coli K12 recA). Plasmid DNA was isolated from tetracycline resistant tranformants and these were screened by digestion with SacI and SmaI. One plasmid which had a SacI site and a SmaI site was called pEVX4.

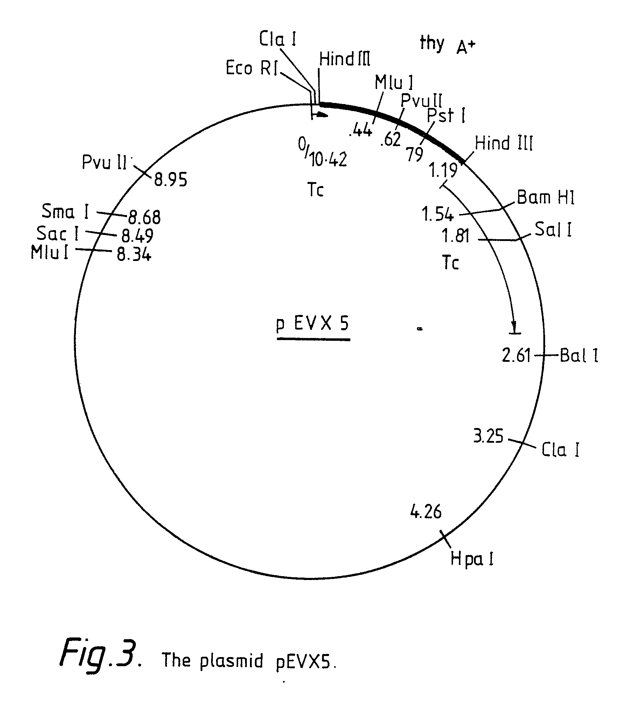

In order to inactivate the tetracycline resistance of pEVX4 and introduce the thyA+ marker into it, plasmid pEVX4 was digested with HindIII and ligated to the 1.2 kb Hind III thyA+ fragment from pBTAH. The DNA was transformed into strain EX98 and thyA+ transformants were selected. Examination of the plasmid content of sevral transformants showed each to have an identical plasmid, and one of these was called pEVX5. This plasmid is shown in Figure 3.

Plasmids PEVX8 and 9 were made as follows:

We wished to make a Plasmid expressing two Vibrio cholerae antigens to allow the simultaneous expression of both antigens on the surface of a bacterial cell. This plasmid may have certain advantages in the construction of a vaccine strain.

The plasmid, pEVX1, having thyA+ as a selection marker, and expressing the OmpV protein, was used. This plasmid has a unique SacI site located at the end of the ompV gene, in the transcription termination region. Plasmid pEVX1 was digested with SacI and ligated to SacI digested pEVX7 DNA. pEVX7 is pUC18 with a 19.5 kb SacI fragment from pPM1002 and has the genes encoding the biosynthesis of the O-antigen from Vibrio cholerae017. The ligated DNA was transformed into strain EX98, thy+ transformants were selected and screened for plasmids with a 19.5 kb SacI insert.

Two such plasmids having the 19.5 kb SacI in opposite orientation relative to each other were called pEVX8 and pEVX9. The strains harbouring these plasmids express both the Vibrio cholerae OmpV protein and the O-antigen and have thyA⁺ as a positive selection marker.

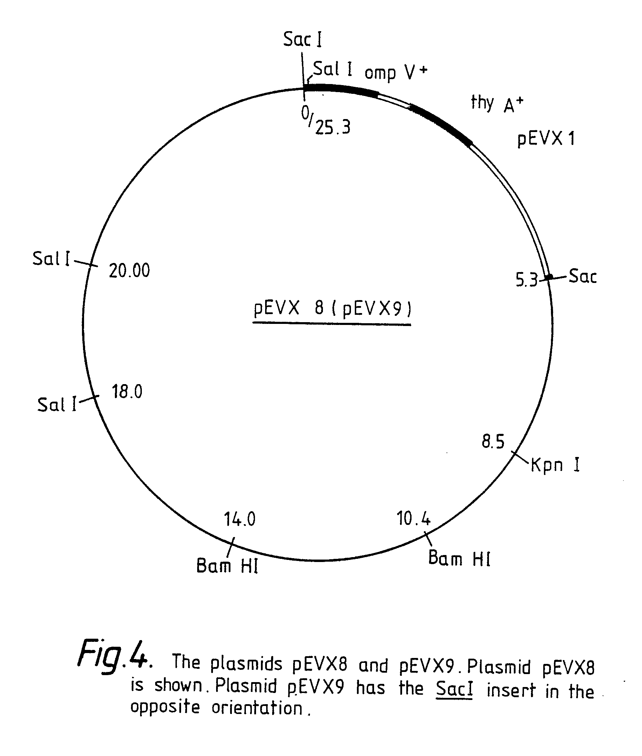

These plasmids are shown in Figure 4.

In a still further aspect of the present invention there is provided a method for preparing a bacterial strain including a non-antibiotic marker, which method includes

(a) providing

(i) a sample of a preselected bacterial strain, and

(ii) a plasmid including a non-reverting thyA⁺ gene as a non-antibiotic marker as described above,

(b) selecting mutants of the preselected bacterial strain defective in the thyA⁺ gene.

(c) complementing the thymine-dependent mutants of the preselected bacterial strain selected with the cloned thyA⁺ gene in said plasmid. The method may further include

(d) selecting the thyA⁺ clone of the preselected bacterial strain so formed on minimal media.

The minimal media may include no thymine. The thyA selection step (b) may include growing the preselected bacterial strain on suitable media in the presence of high levels of thymine.

The suitable media may include an antifolate. The antibiotics may include trimethoprim or aminopterin.

It will be understood that the selection of the thyA⁺ gene as the non-antibiotic selection marker provides particular advantages. Specifically, in the selection step (b) of the method described above, mutants defective in the thyA ⁺ gene are easily selected on media containing antibiotics such as trimethoprim or aminopterin in the presence of high levels of thymine. Such mutants require high levels of thymine for adequate growth on such media. Secondly, the thyA⁺ system does not alter the phenotype of the host bacterial strain. Thirdly, there is likely to be selection in vivo for retention of the plasmid since it is likely that there is insufficient free thymine available for growth of a thymine dependent bacterial organism. Thus, when the selection of the thyA⁺ clones is undertaken on minimal media, that is containing no thymine, only the thyA⁺ clone can be grown and maintained on this media. If the plasmid clone is inherently stable, then large scale propagation of the clone can subsequently be performed in any desired media. If the growth media is deficient in thymine, then selection for the clone is maintained.

The preselected bacterial strain for complementing with the thyA⁺ gene, may be any suitable enteric bacterium. A Salmonella strain or Vibrio strain may be utilised. Trimethoprim resistant derivatives of Salmonella strains may be utilised. Trimethoprim resistant derivatives of Salmonella typhimurium G30 and Salmonella typhi Ty21a have been found to be suitable. A strain of Vibrio cholerae may be utilised.

It will be understood that the bacterial strains so formed may be utilised as live oral vaccines. In this case, the plasmid including the fragment containing the thyA⁺ gene further includes a fragment containing a gene determinant of a disease of interest, such as ompV or the O-antigen as discussed above.

## EXAMPLES

Growth media: Bacterial cells were generally grown in one of the following media:

(a) Nutrient broth, which contained 19 g/l Lab Lemco (Oxoid), 10g/l Peptone (Oxoid) and 5g/l sodium chloride.

(b) Luria Broth, which contained 10g/l Bacto-tryptone (Difco), 5g/l Bacto-yeast extract (Difco), and 5g/l sodium chloride.

(c) Difco nutrient broth, which contained 16g/l nutrient broth (Difco) and 5g/1 sodium chloride.

(d) M9 minimal salts medium, which contained M9 salts, 0.5% glucose, and 0.5% casamino acids (Difco).

Media were solidified by the addition of agar (Oxoid) to 1.5% w/v. Tetracycline was added to media at 8ug/ml, when required. Thymine was added to media at 50 ug/ml for growth and maintenance of thyA mutants.

All incubations were at 37°C.

Selection of thyA mutants: Mutant strains requiring a high level of thymine growth, (i.e. thyA type) were selected as described below.

The bacterial strain was grown overnight in nutrient broth supplemented with thymine. A portion of this culture (0.1 ml to 0.25 ml) was then plated on M9 minimal salts medium containing 50 ug/ml thymine and 25 ug/ml trimethoprim (Sigma). After 24-28 hours incubation, several colonies which grew on this medium were streak purified on the selection medium and tested for their ability to grow on media with or without the addition of thymine. When possible, non-reverting thyA mutant strains were chosen.

Transformation : The method used to transform thyA⁺ plasmids into thyA mutant strains is as follows: The strain was diluted into nutrient broth containing thymine and grown to mid-logarithmic phase. The cells were collected by centrifugation, resuspended in 0.5 volume of cold 100mM MgCl₂, centrifuged again and finally resuspended in 0.1 volume of cold 100mM CaCl₂. After one hour on ice, 0.2 ml cells were added to 0.1 ml plasmid DNA in 100 µl TE (10 mM Tris-HCl, pH7.5, 1mM EDTA). The cells and DNA were incubated on ice for 30 minutes, then heat shocked at 42°C for 2 minutes. Nutrient broth (3 ml) was added, and after incubation for one hour at 37°C, the cells were collected by centrifugation, washed once with M9 minimal salts (without glucose or casamino acids) and resuspended in the same medium. Cells were then plated on M9 minimal salts medium to select thyA⁺ thymine independent) transformants.

## Preparation of plasmid DNA

Two similar methods were employed for the isolation of plasmid DNA: small scale (10 ml) for rapid screening of clones, and a large scale (500-1000 ml) for the preparation of cloning vectors and the final plasmid constructs. Both methods involve cell lysis by freeze thawing, and addition of lysozyme, EDTA and Triton X-100, followed by centrifugation to give a cleared lysate. In the small scale method the lysate is phenolized, precipitated and dried. In the large scale method, the lysate is subjected to CsCl - Ethidium Bromide centrifugation to isolate plasmid DNA.

## Isolation of the 1.2 kb thyA⁺ fragment from pBTAH:

The plasmid pBTAH was isolated from strain RUE10 (pBTAH) provided by Dr. Merlene Belford. 100 ug of this plasmid was digested to completion with HindIII. The 1.2 kb thyA ⁺ fragment was separated from the vector by electrophoresis on a 1% w/v agarose gel. The region of the agarose gel containing this fragment was excised from the gel, placed in a dialysis bag with 4 ml. water and electro-eluted at 100V for three hours. After reversing the direction of elution for 2 minutes, the liquid in the bag was removed and applied to a DE52 (Whatman) column. The column was washed with low salt buffer (0.16m NaCl in 50mM tris -HCl, pH 8.0 and 10mM EDTA). The DNA fragment was then eluted with high salt buffer (as above, but 1M NaCl). The fragment was precipitated with ethanol, collected by centrifugation, washed and dried. It was resuspended in 200 µl of 10mM Tris -HCl, 1 mM EDTA pH 7.4, and used as required.

## DNA manipulations

The DNA methods used were standard. Restriction enzyme digestions were performed essentially as recommended by the enzyme manufacturers, (Pharmacia, New England Biolabs, Boehringer Mannheim).

## Colony Immunoblot

Bacterial cells, grown on nutrient agar, were replica plated onto nitrocellulose filters and lysed in situ according to Henning et al [(1979) Anal. Biochem. 97:153-157]. Positively reacting clones were detected using rabbit antisera against Vibrio cholerae followed by goat anti-(rabbit IgG) coupled to horseradish peroxidase as described by Hawkes et al [(1982) Anal Biochem. 119:142-147].

## Example 1

### Construction of pEVX1

A plasmid which expressed the OmpV protein from Vibrio cholerae and has thyA⁺ as a selection marker was constructed as follows:

An EcoRI-HindIII restriction fragment containing the ompV gene was isolated from plasmid pOmpV210. The ends of the fragment were filled with DNA polymerase (Klenow fragment), and this was ligated with T4 ligase to pBR322 which had been digested with ScaI and had likewise been filled in. Tetracycline resistant transformants were screened with an antiserum for the OmpV protein by an immunoblot. One positive clone was kept and its plasmid called pOmpV500.

The thyA⁺ selection marker was introduced into pOmPV500 by digestion of this Plasmid with HindIII and insertion of the 1.2 HindIII thyA⁺ fragment from plasmid pBTAH. After transformation into strain EX98, thyA⁺ transformants were selected on M9 minimal salts media. The clones obtained had the expected plasmid and still expressed OmpV protein. One such plasmid was called pOmpV501.

A deletion was introduced into pOmpV501 to remove most of the tetracycline resistance gene. The plasmid was digested with NaeI and re-ligated. The transformants obtained (thyA⁺) after transforming the treated plasmid into EX98 were screened for a plasmid smaller than pOmpV501. One deletion plasmid, called pEVX1, had a deletion slightly larger than expected and had lost all its NaeI sites. A map of pEVXI is shown in Figure 1.

Use of pEVX1:

The thyA⁺ selection has proven a useful non-antibiotic selection marker for introducing the ompV gene of Vibrio cholerae into Salmonella typhimurium G30 and Salmonella typhi Ty21a.

thyA mutant derivatives of G30 and Ty21a were isolated as described above. These strains (EX163 and EX164 respectively) were transformed with pEVX1 and selection was made for thyA⁺. (It should be noted that pEVX1 was first passaged by transforming into EX143, a Salmonella typhimurium LT2 strain deficient in all three host restriction systems, before it could be transferred into EX163). The resulting transformants had the correct plasmid and expressed high levels of OmpV protein. The plasmid in both the G30 and the Ty21a derivative seemed to be stably maintained since cultures grown in nutrient broth maintained their thyA⁺ marker as determined by plating on media with and without thymine added.

Example 2

Construction of pEVX17 and pEVX18

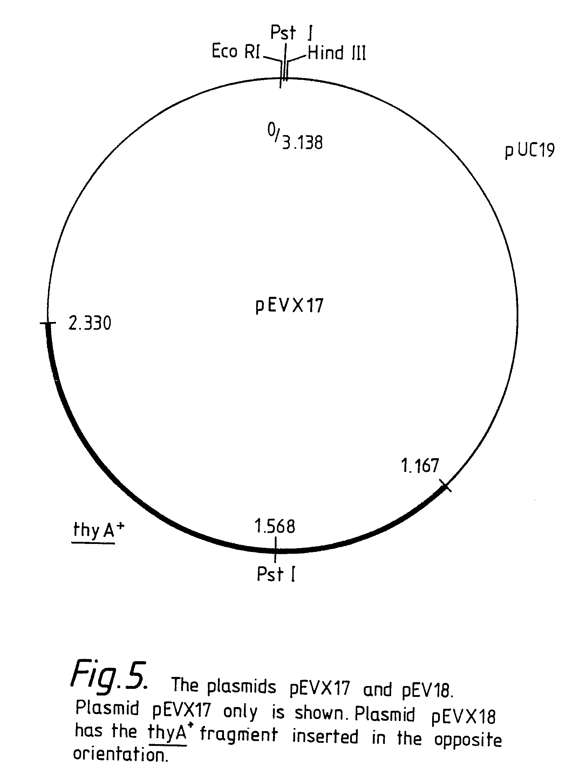

Plasmids pEVX17 and pEVX18 were constructed from plasmid pUC19 by the same procedure used to make pEVX2 and pEVX3 from plasmid pUC18. Hence pEVX17 and pEVX18 (Figure 5), have the thyA⁺ gene inserted in the same orientation as pEVX2 and pEVX3, respectively, but have the polylinker in the opposite orientation.

Example 3

Construction of pEVX23

Plasmid pBTAH can itself be used as a cloning vector. However, to increase the versitality of a pBR322-based thyA⁺ cloning vector, we recloned the 1.12 kb thyA⁺ fragment from pBTAH into the ScaI (located in the ampicillin resistant gene) of pBR322. The resulting plasmid, pEVX23, was constructed as follows: pBR322 was digested to completion with ScaI. This plasmid was then ligated to isolated, end-filled, thyA⁺ HindIII fragment from pBTAH. The ligated DNA was transferred into Ex98 and thyA⁺ transponents were selected on minimal media. The orientation of the thyA⁺ insert was determined by digestion with PstI.

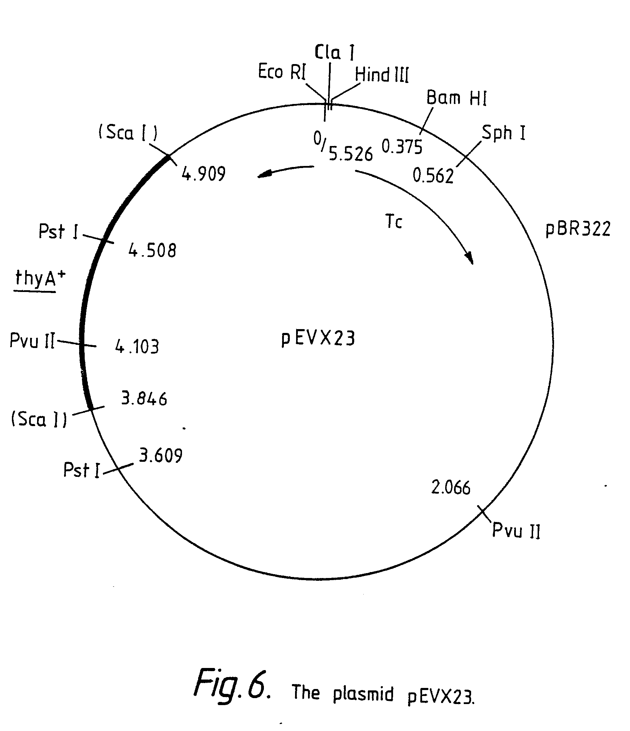

The plasmid from one isolate was called pEVX23 (Figure 6). Strains with this plasmid are sensitive to ampicillin and resistant to tetracycline.

Example 4

Construction of pEVX13 and pEVX14

In addition to the thyA⁺ -Vibrio cholerae O-antigen producing clones already described, several other clones based on pEVX5 were also constructed.

pEVX13 and pEVX14

pEVX5 was digested with Sac I and ligated to SacI digested pEVX7 DNA. The ligated DNA was transformed into strain EX98 and thyA⁺ transformants were selected and screened for plasmids with a 19.5kb Sac I insert. Two such plasmids having the 19.5kb SacI in opposite orientation relative to each other were called pEVX13 and pEVX14. Strains with these plasmids produce Vibrio cholerae O-antigen of the Ogawa-type.

Example 5

Construction of pEVX21 and pEVX22

Plasmids pEVX21 and pEVX22 were constructed from PEVX5 as described above, except that they encode production of Vibrio cholerae O-antigen of the Inaba type. The insert in pEVX21 has the same orientation as that in pEVX14, and that in pEVX22 has the same orientation as that in pEVX13. pEVX13 and pEVX14 were constructed from pEVX20. This plasmid was constructed from Vibrio cholerae 569B (Inaba) and pOmpV500. Strain 569B DNA was digested to completion with SacI. This digested DNA was fractionated on a 5-20% w/v linear sucrose gradient. Fragments of about 20kb were collected and ligated to SacI digested, alkaline phosphatase treated pOmpV500. This ligated DNA was transformed into strain DH1 and tetracycline resistant transformants were selected. Colonies producing Vibrio cholerae O-antigen were detected. The plasmid from one isolate was called pEVX20. This plasmid was used as the source of the 19.5 kb SacI fragment encoding Inaba-specific O-antigen.

Example 6

Construction of pEVX24

As component of our pig scours vaccine, a plasmid encoding K99 production was constructed. Plasmid pFK99 was digested with BamHI, and a fragment encoding K99 fimbriae production was isolated. Plasmid pEVX23 was digested with BamHI, treated with alkaline phosphatase ligated to the K99 fragment. The ligated DNA was transformed into EX98 and thyA⁺ transformants selected. One isolate producing K99 had a plasmid which had the expected structure and was called pEVX24.

## Example 7

### Construction of pEVX25

A second component of our pig scours vaccine is a plasmid encoding K88 production. A 6.5kb Sau3a fragment encoding K88 was isolated from pFM205 and end-filled prior to digestion to Scal digested, alkaline phosphatase treated pBTAH. Subsequent transformation into EX98, selection for thyA⁺ and screening resulted in the isolation of pEVX25.

## Example 8

### Construction of pEVX30 and pEVX31

A third component of our pig scours vaccine is based on plasmids encoding 987P fimbriae. These plasmids were constructed as follows:

Initially a cosmid clone, pEVX26 was isolated which expressed 987P fimbriae. A subclone pEVX26 which expressed 987P fimbriae was isolated by partially digesting pEVX26 with Sau3a and ligating this DNA to BamHI digested pACYC184. One plasmid isolated was called pEVX29. Plasmid pEVX29 was digested with SphI and a SphI fragment was purified. This was end-filled and ligated to Scal digested, alkaline phosphatase treated pBTAH. The digested DNA was transformed into strain EX98 and thyA⁺ transformants were selected. Two isolates expressing 987P were isolated and characterized. In one case, the plasmid was called pEVX30 and had a small deletion (approximately 1kb) at the end of the inserted DNA. The other had a plasmid, pEVX31, of the expected structure.

## Example 9

### Construction of pEVX33 and pEVX37

A fourth component of our pig scours vaccine is based on plasmids encoding F41 fimbriae production. These plasmids were constructed as follows:

Initially, a cosmid clone, pEVX28, which expressed F41 fimbriae was isolated. This plasmid was partially digested with Sau3a and religated to reduce its size. A resulting plasmid was called pEVX32. A 12.3 Kb HindIII fragment was isolated from pEVX32 and ligated to HindIII digested, pEVX23. This DNA was transformed into EX98 and thyA⁺ transformants were selected. One isolate produced F41 fimbriae and had the expected plasmid which was called pEVX33. The size of pEVX33 was reduced by digesting it with ClaI religating and transforming the DNA into EX98. One plasmid having a deletion of the ClaI fragment was called pEVX37.

Finally, it is to be understood that various other modifications and/or alterations may be made without departing from the spirit of the present invention as outlined herein.

## Claims

1. A plasmid bearing a non-antibiotic marker including
    a suitable plasmid cloning vector; and a first cloned fragment of DNA containing a non-reverting thyA⁺ gene attached thereto.

2. A plasmid according to claim 1 wherein the first cloned fragment of DNA is a Hind III-HindIII fragment from the plasmid pBTAH containing a thyA⁺ gene.

3. A plasmid according to claim 1 wherein at least one antibiotic resistance coding region on the plasmid coding vector is inactivated by insertion of the cloned fragment of DNA containing a thyA⁺ gene or is otherwise inactivated.

4. A plasmid according to claim 3 wherein the antibiotic resistance coding region is inactivated by digestion with a suitable enzyme.

5. A plasmid according to claim 4 wherein the at least one antibiotic resistance coding region is an ampicillin and/or a tetracycline resistance coding region.

6. A plasmid according to claim 5 wherein the plasmid cloning vector is selected from the plasmids pSC101, PUC18, pUC19, pBR322 and pBTAH.

7. A plasmid according to claim 2 further including
    at least one further cloned fragment of DNA including a gene determinant of a pathogen of importance in humans and/or animals.

8. A plasmid according to claim 5 wherein the plasmid cloning vector includes an ampicillin resistance coding region which is inactivated by digestion with DraI; and the HindIII-HindIII fragment from the plasmid pBTAH containing a thyA gene is ligated to the digested plasmid cloning vector.

9. A plasmid according to claim 6 wherein the at least one further cloned fragment of DNA includes a gene which expresses an outer membrane protein of Vibrio cholerae and/or a gene which expresses the O-antigen.

10. A plasmid according to claim 9 wherein the at least one further cloned fragment of DNA includes the EcoRI-HindIII fragment from the plasmid pOmpV210.

11. A plasmid according to claim 10 wherein the plasmid cloning vector is pBR322; a HindIII-HindIII fragment from the plasmid PBTAH containing a thyA$^+$ gene is sub-cloned into the HindIII site of the plasmid cloning vector to form the first cloned fragment of DNA; and EcoR1 - HindIII fragment from the plasmid pOmpV210 is sub-cloned to the ScaI site of the plasmid cloning vector to form the further cloned fragment of DNA including a gene determinant of a pathogen ; and wherein the tetracycline resistance coding region on the plasmid is deleted by digestion with NaeI, of the fragments so formed are re-ligated.

12. A plasmid according to claim 1 selected from the plasmid, pEVX1, pEVX2, pEVX3, pEVX5, pEVX8, pEVX9, pEVX13, pEVX14, pEVX17, pEVX18, PEVX21, pEVX22, pEVX23, pEVX24, pEvX25, pEVX30, pEVX31, pEVX33 and pEVX37 as hereinbefore described.

13. A method for preparing a plasmid bearing a non-antibiotic marker including
providing
a first cloned fragment of DNA containing a thyA$^+$ gene; and
a suitable plasmid cloning vector; and
attaching the thyA$^+$ gene fragment to a suitable site on the plasmid such that the thyA$^+$ gene is non-reverting cloning vector.

14. A method according to claim 13 wherein the plasmid cloning vector includes at least one antibiotic resistance coding region and the method further includes
treating the plasmid bearing the thyA$^+$ gene to inactivate at least one antibiotic resistance coding region.

15. A method according to claim 14 wherein the at least one antibiotic resistance is a tetracycline and/or a ampicillin resistance coding region.

16. A method according to claim 15 wherein the second and third steps are combined by inserting the thyA $^+$ gene fragment into the plasmid cloning vector at a site within at least one antibiotic resistance coding region to eliminate the antibiotic resistance.

17. A method for preparing a bacterial strain bearing a non-antibiotic marker, which method includes
providing

a sample of a preselected bacterial strain;.and
a plasmid bearing a non-antibiotic member including a suitable plasmid cloning vector; and
a first cloned fragment of DNA containing a non-reverting thyA$^+$ gene attached thereto;
selecting mutants of the preselected bacterial strain defective in the thyA$^+$ gene;
complementing the thymine-dependent mutants of the preselected bacterial strain selected with the cloned thyA$^+$ gene in said plasmid; and
selecting the thyA$^+$ clone of the preselected bacterial strain so formed on minimal media.

18. A method according to claim 17 wherein the bacterial strain is a Salmonella strain or Vibrio strain.

*Fig.1.* The plasmid pEVX1.

Fig. 2. The plasmids pEVX2 and pEVX3. Plasmid pEVX2 is shown. Plasmid pEVX3 has the thy A gene in the opposite orientation.

*Fig.3.* The plasmid pEVX5.

**Fig.4.** The plasmids pEVX8 and pEVX9. Plasmid pEVX8 is shown. Plasmid pEVX9 has the <u>SacI</u> insert in the opposite orientation.

*Fig.5.* The plasmids pEVX17 and pEV18. Plasmid pEVX17 only is shown. Plasmid pEVX18 has the thyA⁺ fragment inserted in the opposite orientation.

*Fig.6.* The plasmid pEVX23.